# EUROPEAN PATENT SPECIFICATION

(11) **EP 4 351 489 B1**
(45) Date of publication and mention of the grant of the patent: **29.07.2026**
(21) Application number: 22738127.4
(22) Date of filing: 07.06.2022
(51) Int. Cl.: A61F 5/453, A61F 5/455

(54) **FLUID COLLECTION SYSTEMS INCLUDING AT LEAST ONE POROUS CARTRIDGE**
FLÜSSIGKEITSSAMMELSYSTEME MIT MINDESTENS EINER PORÖSEN KARTUSCHE UND EIN VERFAHREN ZUR VERWENDUNG EINES FLÜSSIGKEITSSAMMELSYSTEMS
SYSTÈMES DE COLLECTE DE FLUIDE COMPRENANT AU MOINS UNE CARTOUCHE POREUSE ET UN PROCÉDÉ D'UTILISATION D'UN SYSTÈME DE COLLECTE DE FLUIDE

(30) Priority: 08.06.2021 US 202163208262 P
(43) Date of publication of application: 17.04.2024
(73) Proprietor: Purewick Corporation, Covington, Georgia 30014-1498 (US)
(72) Inventor: ROBICHAUD, Jonathan, Decatur, Georgia 30032 (US)
(74) Representative: Studio Torta S.p.A.
(86) International application number: PCT/US2022/032424
(87) International publication number: WO 2022/261040

(56) References cited:
- US-A- 3 881 486
- US-A1- 2018 228 642
- US-A1- 2021 113 749

## Description

### BACKGROUND

A person or animal may have limited or impaired mobility so typical urination processes are challenging or impossible. For example, a person may experience or have a disability that impairs mobility. A person may have restricted travel conditions such as those experienced by pilots, drivers, and workers in hazardous areas. Additionally, sometimes bodily fluids collection is needed for monitoring purposes or clinical testing.

Urinary catheters, such as a Foley catheter, can address some of these circumstances, such as incontinence. Unfortunately, urinary catheters can be uncomfortable, painful, and can lead to complications, such as infections. Additionally, bed pans, which are receptacles used for the toileting of bedridden individuals are sometimes used. However, bedpans can be prone to discomfort, spills, and other hygiene issues.

Examples of fluid collection systems are disclosed in US3881486A, US2018228642A1.

### SUMMARY

The invention is set out in the appended set of claims.

Embodiments are directed towards fluid collection systems that includes at least one porous cartridge, fluid collection assemblies configured to receive the at least one porous cartridge, and methods of using the same. In an embodiment, a fluid collection system is disclosed. The fluid collection system includes a fluid collection assembly. The fluid collection assembly includes a body including a proximal end region, a distal end region opposite the proximal end region, a front side, and a back side opposite the at least one front surface. The fluid collection assembly includes a connector piece configured to be attached to or integrally formed with the body. The connector piece is positioned closer to the distal end region of the body than the proximal end region. The connector piece defines a conduit connector configured to be connected to a conduit and an arm connector. The conduit connector is positioned adjacent to the back side of the body and the arm connector positioned adjacent to the front side of the body. The fluid collection system also includes an arm including one or more arm walls defining a cavity and an arm opening configured to be in fluid communication with the arm connector. The one or more arm walls defines one or more perforations extending therethrough that allow fluids to flow from an exterior of the arm to the cavity.

In an embodiment, a method of using a fluid collection system is disclosed. The method includes positioning a first porous cartridge adjacent to a urethral opening of an individual. The first porous cartridge including at least one porous material defining a hollowed region. The first porous cartridge reversibly attached to a fluid collection assembly. The fluid collection assembly comprising a body including a proximal end region, a distal end region opposite the proximal end region and closer to a gluteal cleft of the individual than the proximal end region, a front side generally facing a vaginal region of the individual, and a back side opposite the at least one front surface. The fluid collection assembly also includes a connector piece attached to or integrally formed with the body. The connector piece is positioned closer to the distal end region of the body than the proximal end region. The connector piece defines a conduit connector configured to be connected to a conduit and an arm connector. The conduit connector is positioned on the back side of the body and the arm connector positioned on the front side of the body. One of the first porous cartridge or the fluid collection assembly includes an arm including one or more walls defining a cavity and an opening that is in fluid communication with the arm connector. The one or more walls define one or more perforations extending therethrough that allow fluids to flow from an exterior of the arm to the cavity. At least a portion of the arm is disposed in the hollowed region of the first porous cartridge. The method also includes receiving one or more bodily fluids from the vaginal region of the individual into the at least one porous material of the first porous cartridge and flowing at least some of the one or more bodily fluids received into the at least one porous material of the first porous cartridge into the cavity of the arm.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIGS. 1A-1C** are an isometric front, isometric back, and top plan views of a fluid collection system, according to an embodiment.
**FIG. 1D** is a cross-sectional schematic of the fluid collection system taken along plane 1D-1D shown in **FIG. 1A****.**
**FIG. 1E** is an exploded view of the fluid collection system.
**FIGS. 2A** to **2C** are isometric back, isometric front, and back plan views of a fluid collection system, according to an embodiment.
**FIG. 2D** is a cross-sectional view of the fluid collection system taken along plane 2D-2D illustrated in **FIG. 2A****.**
**FIG. 2E** is an exploded view of the fluid collection system.
**FIG. 3** is a partially exploded view of a fluid collection system illustrating the fluid collection assembly and the porous cartridge spaced from each other, according to an embodiment.
**FIG. 4** is a partially exploded view of a fluid collection system illustrating the fluid collection assembly and the porous cartridge spaced from each other, according to an embodiment.
**FIG. 5** is a partially exploded view of a fluid collection system including one or more anchors, according to an embodiment.
**FIG. 6** is a cross-sectional view of a fluid collection system taken perpendicularly to a longitudinal axis thereof, according to an embodiment.
**FIG. 7** is a side plan view of a portion of a fluid collection system that includes a plurality of porous cartridges that include different lateral dimensions, according to an embodiment.
**FIG. 8** is a side plan view of a portion of a fluid collection system that includes a plurality of porous cartridges that include different lengths, according to an embodiment.
**FIG. 9** is a block diagram of a fluid collection system for fluid collection, according to an embodiment.

### DETAILED DESCRIPTION

Embodiments are directed towards fluid collection systems that include at least one porous cartridge, fluid collection assemblies configured to receive the at least one porous cartridge, and methods of using the same. An example fluid collection system includes a fluid collection assembly and at least one porous cartridge configured to be reversibly attached to the fluid collection assembly (*i.e.,* attached and detached substantially without damaging the fluid collection assembly and the porous cartridge). The fluid collection assembly includes a body and a connector piece integrally formed with, attached to, or attachable to the body. The connector piece includes a conduit connector configured to be connected to at least one conduit and an arm connector. The fluid collection system also includes an arm that forms part of the porous cartridge or the fluid collection assembly. The arm is configured to be connected or otherwise in fluid communication with the arm connector of the connector piece. The arm includes one or more arm walls that define an arm opening at one end, a cavity extending through at least a portion thereof, and one or more perforations extending through the arm walls that allow one or more bodily fluids to flow from an exterior of the arm into the cavity. The porous cartridge includes a porous material defining a hollowed region. The hollow region is configured to receive the arm of the fluid collection assembly. The porous cartridge is configured to be reversibly attached to the fluid collection assembly.

During use, the fluid collection system may be positioned such that the porous cartridge is positioned adjacent to a vaginal region of an individual. For example, at least a portion of the porous cartridge is positioned between the labia folds of the individual such that the porous cartridge is adjacent to or abuts the urethral opening of the individual. After positioning the fluid collection system, the porous cartridge may receive one or more bodily fluids (*e.g.,* urine, blood, sweat, etc.) from the urethral opening of the individual or, more generally, the vaginal region. At least some of the bodily fluids may flow through the porous cartridge (*e*.*g*., flow into the porous material and into the cavity of the arm through the one or more perforations formed in the arm). The bodily fluids may flow through the cavity of the arm, through the connector piece, and out through the conduit via the arm connector and the conduit connector. Thus, the bodily fluids discharged by the individual may be removed from the individual thereby maintaining the individual dry and comfortable. The porous cartridge may be removed from the fluid collection assembly after at least some of the bodily fluids are removed from the porous cartridge. Optionally, a new porous cartridge may be attached to the fluid collection assembly after the original porous cartridge is removed thereby allowing the fluid collection assembly to be reused.

In an embodiment, the fluid collection assembly includes a fluid storage container and a vacuum source. The fluid storage container and the vacuum source may be in fluid communication (*e*.*g*., via one or more conduits) with the fluid collection assembly and the porous cartridge. For example, a vacuum source may be in fluid communication with the fluid collection assembly and the porous cartridge. The vacuum source may be configured to apply a vacuum to the arm and the porous cartridge. The vacuum may remove the bodily fluids received by the porous cartridge faster than if wicking (*e*.*g*., capillary action) and gravity are used to remove the bodily fluids. The bodily fluids removed from the porous cartridge may be disposed in the fluid storage container.

The fluid collection systems disclosed herein are improvements over conventional fluid collection assemblies for one or more reasons. In an example, conventional fluid collection systems include a porous material that is not removable from a fluid impermeable body. Such conventional fluid collection systems may not be environmentally sustainable (*e*.*g*., generate excessive waste). Other conventional fluid collection systems may allow for the porous material to be removed therefrom by bending a fluid impermeable body that the porous material is disposed within. However, such bending may make removing the porous material complicated, may result in compressing the porous material thus releasing at least some of the bodily fluids held therein, or may damage the fluid impermeable body. The fluid collection systems disclosed herein remedy these issues by providing a porous cartridge that may be easily removed from the fluid collection assembly without or substantially without bending any other component of the fluid collection assembly. As such, the fluid collection systems disclosed herein decrease waste, allow the fluid collection assemblies of such systems to be reused *(e.g.,* after washing), prevent bodily fluids being released from the porous cartridge, prevent damage to other components thereof, and may be easily used. The fluid collection systems disclosed herein may include other improvements over conventional fluid collection systems, as discussed in more detail below.

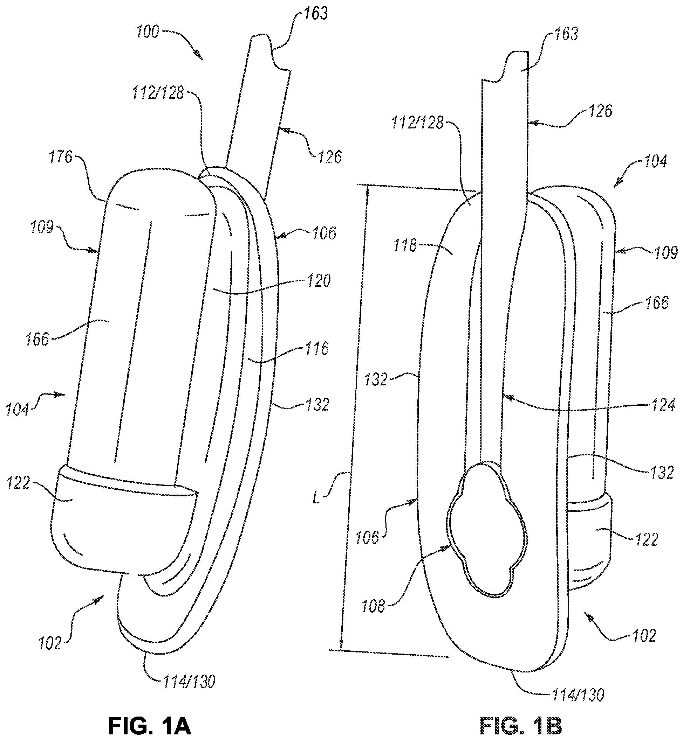

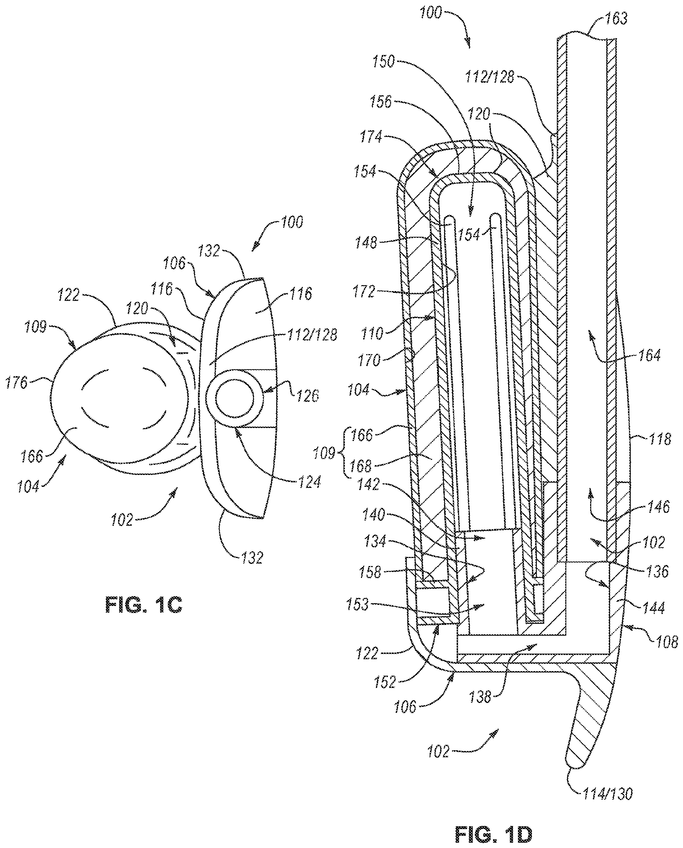

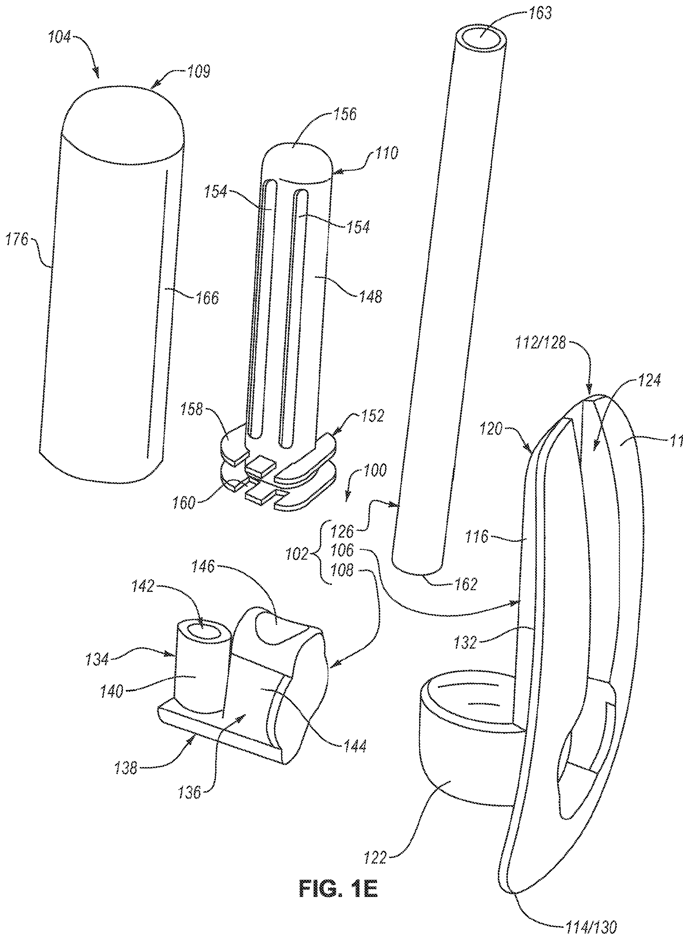

**FIGS. 1A** to **1C** are an isometric front, isometric back, and top plan views of a fluid collection system 100, according to an embodiment. **FIG. 1D** is a cross-sectional schematic of the fluid collection system 100 taken along plane 1D-1D shown in **FIG. 1A****.** **FIG. 1E** is an exploded view of the fluid collection system 100. In the illustrated embodiment, the fluid collection system 100 includes a fluid collection assembly 102 and a porous cartridge 104. The fluid collection assembly 102 includes a body 106 and a connector piece 108. The porous cartridge 104 includes at least one porous material 109. The fluid collection system 100 also includes an arm 110 that may form part of the fluid collection assembly 102 or the porous cartridge 108. The porous material 107 is configured to be disposed on the arm 110. The porous cartridge 104 is configured to be reversibly attached to the fluid collection assembly 102.

The body 106 of the fluid collection assembly 102 includes a proximal end region 112, a distal end region 114 opposite the proximal end region 112, a front side 116, and a back side 118 opposite the front side 116. Generally, during use, the distal end region 114 is closer to the gluteal cleft of the individual than the proximal end region 112 and the front side 116 generally faces the vaginal region of the individual. The body 106 may be formed from silicone, neoprene, a thermoplastic elastomer, another fluid impermeable material, or combinations thereof.

The body 106 may include a central portion 120. In an embodiment, the central portion 120 includes portions of the body 106, other than the sump 122, that are configured to at least one of contact (*e*.*g*., support) a portion of the porous cartridge 104. In such an embodiment, the central portion 120 of the body 106 may include a bulge that is configured to contact the porous cartridge 104. The bulge may extend outwardly from the front surface 115 of the body 106. The bulge may limit the amount of contact between the porous cartridge 104 and the body 106 such that the body 106 does not significantly limit which portions of the porous cartridge 104 may contact the vaginal region of the individual while also providing support to the porous cartridge 104. In an embodiment, the central portion 120 may include portions of the body 106, other than the sump 122, that are configured to be attached to or integrally formed with the connector piece 108.

In an embodiment, the body 106 includes one or more flanges extending from the central portion 120 and/or the sump 122. The flanges may prevent or at least inhibit turning of the fluid collection assembly 102 during use. The flanges may also provide more locations for underwear or other clothing to contact and press against the fluid collection assembly 102 which may facilitate securing the fluid collection system 100 to the vaginal region of the individual. The flanges may additionally increase the width of the fluid collection assembly 102 sufficiently that the thighs of the individual may contact the flanges. Contacting the thighs of the individual against the flanges may facilitate securement of the fluid collection assembly 102 to the individual. The flanges may also contact the labia majora of the individual depending on the size of the labia majora of the individual. Contacting the flanges to the labia majora may further inhibit turning of the fluid collection assembly 102 during use and may improve patient comfort.

The flanges may include any portion of the body 106 that are distinct from the central portion 120 of the body 106 and the sump 122. In other words, the flanges may extend laterally outwardly from the central portion 120 and the body 106. The flanges may be distinguishable from the central portion 120 because the flanges at least one of exhibit a thickness that is less than the central portion 120, do not form part of the bulge, do not form part of the recess 124 that receives the conduit 126, and are not attached to the connector piece 108. In an embodiment, the flanges may include at least one of an upper flange 128 forming the proximal end region 112, a bottom flange 130 opposite the upper flange 128 that forms the distal end region 114, or one or more lateral flanges 132.

The flanges of the body may extend from the central portion 120 by a distance that is about 1 mm or greater, about 2 mm or greater, about 3 mm or greater, about 4 mm or greater, about 5 mm or greater, about 6 mm or greater, about 7.5 mm or greater, about 1 cm or greater, about 1.25 cm or greater, about 1.5 cm or greater, about 2 cm or greater, about 2.5 cm or greater, about 3 cm or greater, about 4 cm or greater, about 5 cm or greater, or in ranges of about 1 mm to about 3 mm, about 2 mm to about 4 mm, about 3 mm to about 5 mm, about 4 mm to about 6 mm, about 5 mm to about 7.5 mm, about 6 mm to about 1 cm, about 7.5 mm to about 1.25 cm, about 1 cm to about 1.5 cm, about 1.25 cm to about 2 cm, about 1.5 cm to about 2.5 cm, about 2 cm to about 3 cm, about 2.5 cm to about 4 cm, or about 3 cm to about 5. The distance that the flanges extend from the central portion 120 may be selected based on the expected size of the vaginal region of the individual (*e*.*g*., larger flanges are configured to be used with larger vaginal regions) or the expect rotational forces applied to the fluid collection assembly 102 during use. In some examples, at least some of the flanges may extend further from the central portion 120 that other flanges. For instance, as illustrated, the bottom flange 130 may extend further from the central portion 120 than the upper flange 128 since some individuals may find the longer bottom flange 130 more comfortable.

In an embodiment, the one or more flanges may exhibit a concave curve relative to the front side 116 of the body 106. The concave curve of the flanges may extend from the proximal end region 112 to the distal end region 114. The concave curve of the flanges may allow the flanges to better conform to the shape of the vaginal region since the vaginal region is curved. Conforming the flanges to the shape of the vaginal region may make the fluid collection assembly 102 more comfortable by more uniformly distributing pressure across the vaginal region, especially when the flanges contact the labia majora. In an embodiment, the central portion 120 may also exhibit a concave curve relative to the front side 116 of the body 106.

In an embodiment, the body 106 may include a sump 122 at or near the distal end region 114. The sump 122 may extend outwardly from the front side 116 of the body 106. During use, the sump 122 may be configured to be at, near, or otherwise in fluid communication with a gravimetric low point of the porous cartridge 104. For example, the sump 122 may receive a portion of the porous cartridge 104 therein. The sump 122 may receive at least a portion of the arm 110. The sump 122 may prevent or at least inhibit bodily fluids from leaking from the fluid collection assembly 102. The sump 122 may include or be at least partially formed by at least a portion of the connector piece 108 (*e.g.,* the arm connector 134) at least partially disposed therein. The sump 122 directs the bodily fluids either towards the connector piece 108. The connector piece 108 may define one or more perforations therein that is configured to receive bodily fluids present in the sump 122.

In an embodiment, the central portion 120 of the body 106 may define a recess 124 that is configured to receive a conduit 126. The recess 124 may extend from or near the proximal end region 112 to or near the distal end region 114 thereby allowing the conduit 126 to extend from or near the individual's abdominal region to the connector piece 108. The bulge formed by the central portion 120 may facilitate formation of the recess 124 without increasing a thickness of the body 106 which may otherwise decrease the malleability of the body 106. Also, the presence of the recess 124 formed by the central portion 120 may decrease the thickness of the bulge thereby increasing the malleability of the body 106. The increased malleability of the central portion 120 may at least one of make conforming the fluid collection system 100 to the vaginal region easier, make using the fluid collection system 100 more comfortable, or allow the fluid collection assembly 102 to be easier to fit under underwear or other clothing.

In an embodiment, the recess 124 may be configured such that the body 106 encloses and/or abuts less than 50% of a circumference of the conduit 126, thereby allowing the conduit 126 to freely enter and leave the recess 124 during use. Allowing the conduit 126 to freely enter and leave the recess 124 may facilitate positioning of the fluid collection system 100 such that the porous cartridge 104 is adjacent to the vaginal region even when the conduit 126 is bending away from the vaginal region. Also, allowing the conduit 126 to freely enter and leave the recess 124 may increase the likelihood that movement of the conduit 126 does not move the porous cartridge 104 relative to the vaginal region since movement of the porous cartridge 104 may cause leaking. In an embodiment, at least a portion of the recess 124 may be configured such that the body 106 encloses and/or abuts more than 50% (*e.g.,* 51% to about 55%, about 53% to about 57%, or about 55% to about 60%) of the circumference of the conduit 126. Enclosing more than 50% of the circumference of the conduit 126 may more securely attach the conduit 126 to the body 106 and may allow the conduit 126 to provide additional structure to the body 106. The percentage of the conduit 126 enclosed and/or abutted by the body 106 may be selected such that the inherent elasticity of the body 106 and the conduit 126 allows the conduit 126 to be easily snapped into and out of the recess 124. As such, the conduit 126 may be removed from the recess 124 to facilitate positioning the porous cartridge 104 adjacent to the vaginal region or when the conduit 126 is moved.

The body 106 may exhibit a length L measured from the proximal end region 112 to the distal end region 114. The length L of the body 106 may be selected to be about 20 cm or less, about 18 cm or less, about 16 cm or less, about 14 cm or less, about 12 cm or less about 10 cm or less, about 9 cm or less, about 8 cm or less, about 7 cm or less, about 6 cm or less, 5 cm or less, about 4 cm or less, or in ranges of about 3 cm to about 5 cm, about 4 cm to about 6 cm, about 5 cm to about 7 cm, about 6 cm to about 8 cm, about 7 cm to about 9 cm, about 8 cm to about 10 cm, about 9 cm to about 12 cm, about 10 cm to about 14 cm, about 12 cm to about 16 cm, about 14 cm to about 18 cm, or about 16 cm to about 20 cm. Generally, the length of the body 106 is the largest length of the fluid collection assembly 102 and the fluid collection system 100, excluding the conduit 126. Some conventional fluid collection assemblies and the fluid impermeable bodies thereof may exhibit a length that is about 18 cm or greater. However, the structure of the fluid collection system 100 allows the body 106 and, by extension, the fluid collection system 100 as a whole to exhibit a length L that is less than at least some conventional fluid collection assemblies. The decreased length L of the body 106 and the fluid collection system 100 makes the fluid collection system 100 more discrete than the conventional fluid collection assemblies. Further, during use, the decreased length L of the body 106 allows the fluid collection system 100 to be further spaced from the gluteal cleft of the individual which decreases the likelihood that stool migrates from the gluteal cleft to the fluid collection system 100 and that the fluid collection system 100 interferes with stool collection and defecation.

As previously discussed, the fluid collection assembly 102 includes a connector piece 108 that is attached to (*e.g.,* with an adhesive, welding, interference fit, etc.) or integrally formed with the body 106. The connector piece 108 is positioned at or near the distal end region 114 of the body 106 which allows the connector piece 108 to receive bodily fluids that flow to the gravimetric low point of the porous cartridge 104. In an embodiment, a portion of the connector piece 108 may be positioned in the sump 122 of the body 106. In an embodiment, not shown, the connector piece 108 may form the sump 122 instead of the body 106.

The connector piece 108 is configured to be connected to the arm 110 and the conduit 126. As such, the connector piece 108 may include an arm connector 134 configured to be attached to or otherwise in fluid communication with the arm 110 and a conduit connector 136 configured to be attached to or otherwise in fluid communication with the conduit 126. The connector piece 108 is also configured to allow the porous material 109 and the arm 110 to be in fluid communication with the conduit 126 even though the porous material 109 and the arm 110 are on opposite sides of the body 106 than the conduit 126. As such, the connector piece 108 may include a channel 138 *(e.g.,* tube) extending from the arm connector 134 to the conduit connector 136. The channel 138 allows the bodily fluids to flow from the front side 116 of the body 106 *(e.g.,* from at least one of the porous cartridge 104, the arm 110, or the arm connector 134) to the back side 118 of the body 106 (*e.g.,* to at least one of the conduit 126 or the conduit connector 136). The body 106 may define a passageway that allows the connector piece 108 *(e.g.,* the channel 138) to extend from the front side 116 to the back side 118.

The arm connector 134 of the connector piece 108 is positioned adjacent to the front side 116 of the body 106. In an example, the arm connector 134 is at least partially positioned in the sump 122 of the body 106. As previously discussed, the arm connector 134 is configured to be connected to the arm 110. For example, the arm connector 134 may include one or more first connector walls 140 extending upwardly from an adjacent portion of the connector piece 108 (e*.g.,* from the channel 138). The first connector walls 140 may be configured to receive or be received by the arm 110. The first connector walls 140 may define a first connector opening 142 at or near a top portion (*i.e.,* a portion furthest spaced from the channel 138). The first connector opening 142 is configured to receive bodily fluids from the arm 110 and provide a vacuum to the arm 110. The first connector walls 140 may also define one or more perforations (not shown) that are configured to receive one or more bodily fluids that are not received by the arm 110 and are present in the sump 122. Such perforations may be formed at or near a portion of the first connector walls 140 adjacent to the channel 138.

The conduit connector 136 of the connector piece 108 is positioned adjacent to the back side 118 of the body 106. As previously discussed, the conduit connector 136 is configured to be connected to the conduit 126. The conduit connector 136 may include one or more second connector walls 144 extending upwardly from an adjacent portion of the connector piece 108 (*e.g.,* from the channel 138). The second connector walls 144 may be configured to receive or be received by the conduit 126. The second connector walls 144 may define second connector opening 146 at or near a top portion (*i.e*., a portion furthest spaced from the channel 138). The second connector opening 146 is configured to provide bodily fluids received into the connector piece 108 to the conduit 126 and provide a vacuum to the channel 138.

In an embodiment, at least a portion of the connector piece 108 may exhibit a rigidity that is greater than the body 106. The increased rigidity of the connector piece 108 relative to the body 106 may facilitate attachment of the arm 110 and the conduit 126 to the connector piece 108. In an example, the connector piece 108 may exhibit a rigidity that is greater than the body 106 when the connector piece 108 is formed from a material exhibiting at least one of a greater Young's modulus (*i.e.,* modulus of elasticity), yield strength, or ultimate tensile strength than a material that forms the body 106. In an example, the connector piece 108 may exhibit a rigidity that is greater than the body 106 when the connector piece 108 exhibits a thickness that is greater than the body 106.

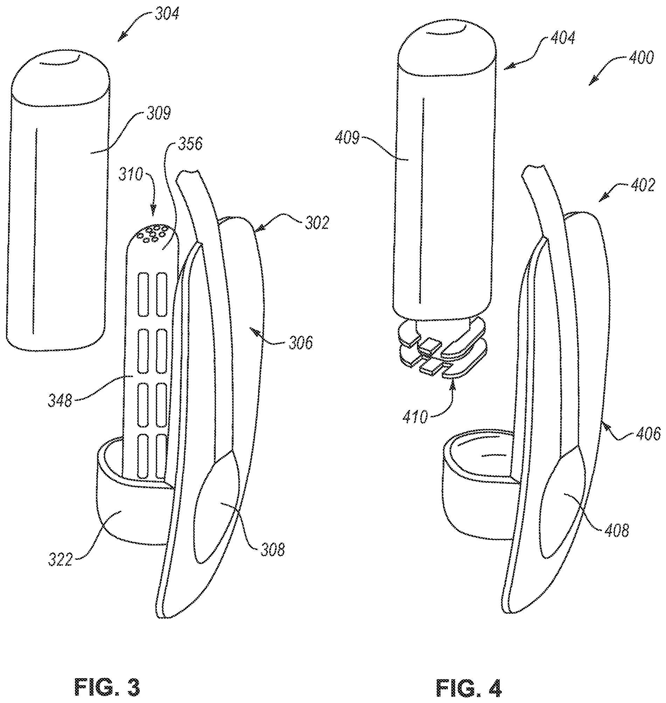

As previously discussed, the fluid collection system 100 102 includes an arm 110. The arm 110 may form part of the fluid collection assembly 102 (as shown in **FIG. 3****)** or part of the porous cartridge 104 (as shown **in** **FIG. 4****).** The arm 110 is configured to provide support to the porous material 109, allow the porous cartridge 104 to be reversibly attached to the fluid collection assembly 102, receive bodily fluids from the porous material 109, and allow bodily fluids received thereby to flow to the connector piece 108 and to the conduit 126. In an embodiment, the arm 110 is reversibly attached to (*e.g.,* via an interference fit), non-reversibly attached to (*e.g.,* with an adhesive, welding, etc.) or integrally formed with the arm connector 134 of the connector piece 108.

The arm 110 of the fluid collection assembly 102 may be an improvement over conventional fluid collection assemblies. For example, conventional fluid collection systems often introduce a vacuum to a portion of the porous material thereof that is spaced from a location of the porous material that contacts or is adjacent to the urethral opening which may result in a weak vacuum at or near the urethral opening. However, the arm 110 of the fluid collection assembly 120 disclosed herein may extend through almost all of the porous material 109 which may facilitate a more uniform vacuum throughout the material 109 or a more targeted vacuum compared to conventional fluid collection assemblies.

The arm 110 allows the porous cartridge 104 to be attached to the fluid collection assembly 102 without positioning most or all of the porous material 109 within a fluid impermeable barrier (*e*.*g*., within the body 106). As such, the arm 110 allows the porous material 109 to exhibit a variety of cross-sectional shapes, widths, and lengths since the cross-sectional shape, width, and length of the porous material 109 are not constrained by the fluid impermeable barrier. The arm 110 may exhibit a cross-sectional shape that is the same as or different than the cross-sectional shape of the porous material 109. The cross-sectional shape of the arm 110 may affect the distribution of the vacuum provided to the porous material 109. For instance, the arm 110 may provide a more uniform vacuum to the porous material 109 when the arm 110 exhibits a cross-sectional shape that is the same as the cross-sectional shape of the porous material 109. However, the arm 110 may provide a more targeted vacuum when the arm 110 exhibits a cross-sectional shape that is different than the cross-sectional shape of the porous material 109. In an example, the arm 110 may exhibit a generally circular cross-sectional shape, a cross-sectional shape including at least one apex (*e.g.,* a generally triangular cross-sectional shape or a generally circular cross-sectional shape having an edge pitched to form the apex), or any other suitable cross-sectional shape.

The arm 110 provides support to the porous material 109. In some examples, the arm 110 may be flexible (*e*.*g*., formed from a compliant material) such that the porous material 109 may be bent to exhibit a shape that corresponds to the shape of the vaginal region. Bending the porous material 109 to exhibit a shape that corresponds to the shape of the vaginal region may minimize leakage of bodily fluids. Examples of materials that may form a compliant arm 110 may include high-density polyethylene or low-density polyethylene.

The arm 110 may include one or more arm walls 148. The arm walls 148 define a bottom portion 152. The bottom portion 152 is configured to attach the arm 110 to the arm connector 134 of the connector piece 108. The bottom portion 152 may also define at least one arm opening 153 that allows the arm 110 to be in fluid communication with the connector piece 108. For example, the bottom portion 152 is configured to receive or be positioned within the arm connector 134 such that bodily fluids flowing through the bottom portion 152 and out the arm opening 152 flow into the connector piece 108. The arm walls 148 also define a cavity 150 extending upwardly from the arm opening 135 and one or more perforations 154 extending through the arm walls 148. The perforations 154 allow bodily fluids in the porous material 109 to flow through the arm walls 148 and be received into the cavity 150. The bodily fluids received into the cavity 150 may flow to the arm opening 153 and into the connector piece 108. The bottom portion 152, the cavity 152, and the perforations 154 also allow a vacuum to be provided to the porous material 109.

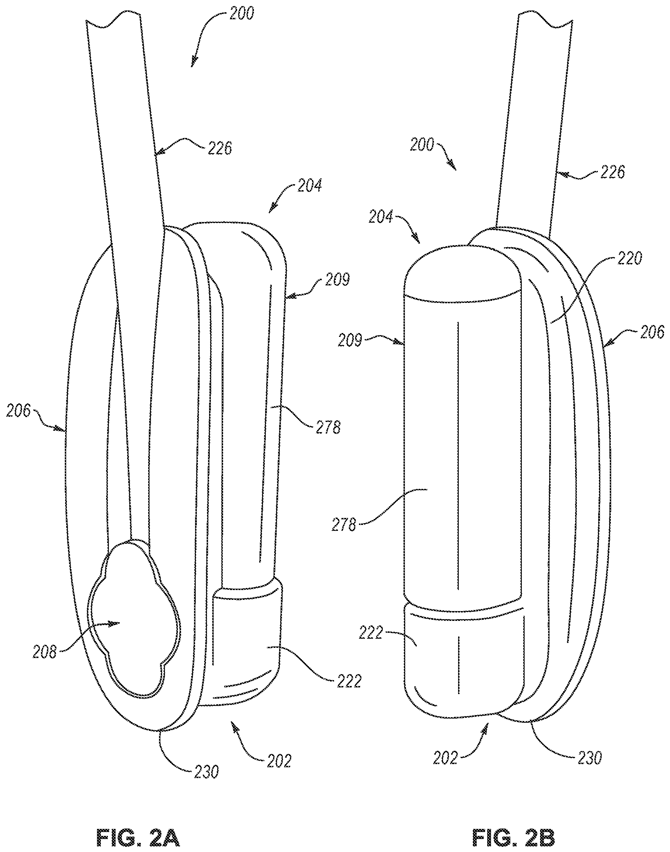

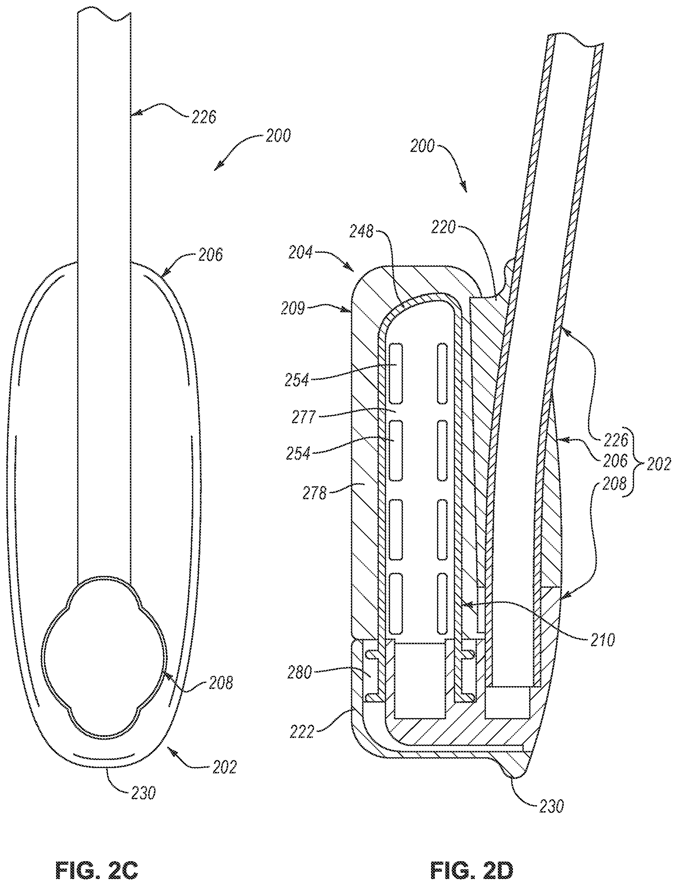

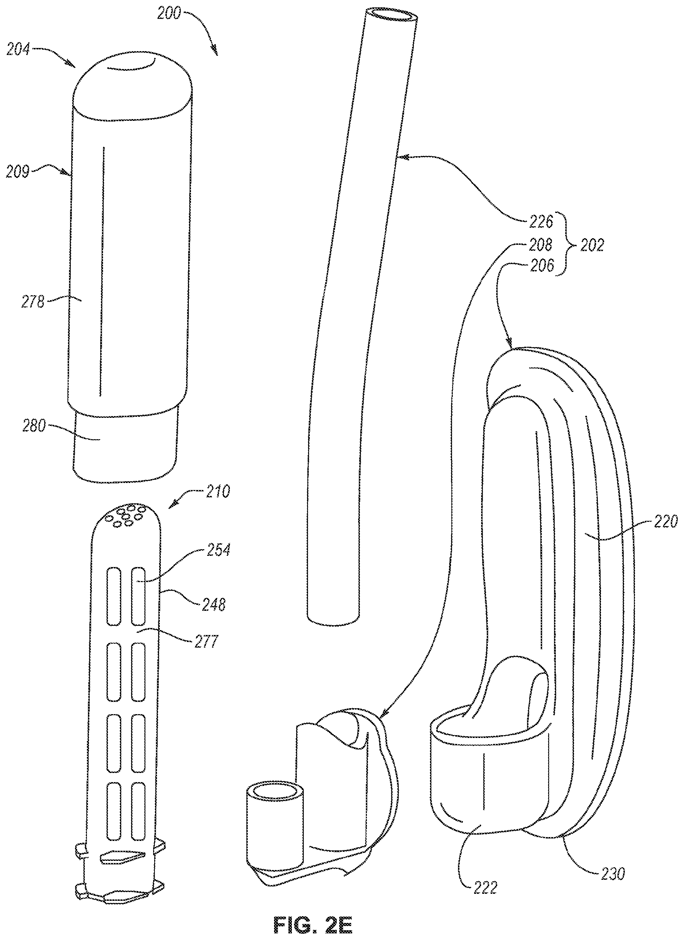

The location of the perforations 154 dictate how the vacuum is provided to the porous material 109. In an example, the perforations 154 may extend from or near the bottom portion 152 to or near a top side 156 of the arm 110 (*i.e.,* a side of the arm 110 opposite the bottom portion 152) which may allow the vacuum to be provided substantially uniformly along the length of the porous material 109, unlike some conventional fluid collection assemblies that introduce a vacuum near a bottom of the porous material thereof. In an example, the perforations 154 may be positioned on the top side 156 of the arm 110 thereby ensuring that the vacuum is provided to the adjacent portions of the porous material 109 (*see* **FIG. 2E****).** In an embodiment, the surface area density of the perforations 154 may be generally uniform thereby causing the vacuum to be provided more uniformly throughout the porous material 109. In an embodiment, the surface area density of the perforations 154 may be non-uniform thereby causing the vacuum to be more preferentially provided to certain locations of the porous material 109, such as portions of the porous material 109 expected to receive more bodily fluids that other portions. Preferentially providing the vacuum to certain locations of the porous material 109 may increase the removal rate of bodily fluids from these portions of the porous material 109. In an example, the surface area density of the perforations 154 may be greater on a portion of the arm walls 148 that are further spaced from the front side 116 of the body 106 than portions of the arm walls 148 that are closer to the front side 116 since the bodily fluids are more likely to be received by portions of the porous material 109 that are further spaced from the front side 116. In an example, the surface area density of the perforations 154 at or near the top side 156 (*e.g.,* a top half of the arm 110) may be greater than the surface area density of the perforations 154 at or near the bottom portion 152 (*e.g.,* a bottom half of the arm 110) since the urethral opening is generally positioned adjacent to a top half of the porous material 109.

In an embodiment, as shown, the perforations 154 may include one or more elongated slits extending along at least half of the length of the arm 110. In an embodiment, the perforations 154 may include generally circular cutouts formed in the arm walls 148.

In an embodiment, the arm 110 may include one or more arm flanges 158 extending from the arm walls 148 of the bottom portion 152. The arm flanges 158 may prevent over insertion of the porous material 109104 on the arm 110 which would otherwise damage the porous material 109 (*e.g.,* cause the arm 110 to poke through the porous material 109). The arm flanges 158 may also provide support to the sump 122 and/or cause the arm 110 to exhibit an interference fit with the sump 122. In an example, the arm flanges 158 may define one or more flange perforations 160 which allows bodily fluids to flow through the arm flanges 158 to a gravimetric low point of the sump 122. The bodily fluids may then be removed from the sump 122 via one or more perforations formed in the arm connector 134 or in the arm walls 148 adjacent to the bottom portion 152.

As previously discussed, in an embodiment, the arm 110 may not form part of the porous cartridge 104 and, instead, the arm 110 forms part of the fluid collection assembly 102. In such an embodiment, the arm 110 is attached to the body 106 (*e.g.,* the sump 122). The porous cartridge 104 may be reversibly attached to the fluid collection assembly 102 by sliding the porous material 109 along the arm 110. Detaching the porous cartridge 104 from the fluid collection assembly 102 may include sliding the porous material 109 away from the arm 110. In an embodiment, the arm 110 does not form part of the fluid collection assembly 102 and, instead, the arm 110 forms part of the disposable cartridge that is attached to the fluid collection assembly 102. In such an embodiment, the arm 110 is attached to the porous cartridge 104. Attaching the porous cartridge 104 to the fluid collection assembly 102 includes attaching the arm 110 to the arm connector 134. Detaching the porous cartridge 104 from the fluid collection assembly 102 includes detaching the arm 110 from the arm connector 134. It is noted that including the arm 110 in the disposable cartridge increases the waste generated while using the fluid collection system 100 but decreases the surface area of the fluid collection assembly 102 that may need to be cleaned.

As previously discussed, the fluid collection assembly 102 may include the conduit 126. The conduit 126 may be used to remove the bodily fluids from the connector piece 108. The conduit 126 includes at least one wall defining an inlet 162, an outlet 163 downstream from the inlet 162, and a passageway 164. The inlet 162 may be attached to or otherwise in fluid communication with the conduit connector 136. For example, the inlet 162 may receive or be receive by the conduit connector 136. The outlet 163 of the conduit 126 may be operably coupled to a vacuum source, such as a vacuum pump.

As previously discussed, the porous cartridge 104 includes at least one porous material 109. As shown in **FIG. 1D****,** the porous material 109 includes at least one of a fluid permeable membrane 166 and a fluid permeable support 168. In an embodiment, the porous material 109 may be configured to wick any bodily fluids away from an exterior thereof towards the arm 110. The permeable properties referred to herein may be wicking, capillary action, diffusion, or other similar properties or processes, and are referred to herein as "permeable" and/or "wicking." Such "wicking" and/or "permeable" properties may not include absorption of the bodily fluids into at least a portion of the porous material 109, such as not include adsorption of the bodily fluids into the fluid permeable support 168. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the porous material 109 (*e.g.,* absorbency), such as less than about 30 wt% of the dry weight of the porous cartridge 104, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the porous cartridge 104. In an embodiment, the porous material 109 may include at least one absorbent or adsorbent material.

In an embodiment, the porous material 109 may include the fluid permeable membrane 166 disposed on an exterior surface 170 of the fluid permeable support 168. The fluid permeable membrane 166 may cover at least a portion (*e.g.,* all) of the exterior surface 170. The fluid permeable membrane 166 may be composed to wick the bodily fluids away from an exterior of the porous material 109 (*e.g.,* from a vaginal region, such as from a urethral opening), thereby preventing the bodily fluids from escaping the porous material 109.

In an embodiment, the fluid permeable membrane 166 may include any material that may wick the bodily fluids. For example, the fluid permeable membrane 166 may include fabric, such as a gauze *(e.g.,* a silk, linen, or cotton gauze), another soft fabric, another smooth fabric, a nonwoven material, or any of the other porous materials disclosed herein. Forming the fluid permeable membrane 166 from gauze, soft fabric, and/or smooth fabric may reduce chaffing caused by the porous material 109.

The porous material 109 may include the fluid permeable support 168. The fluid permeable support 168 is configured to support the fluid permeable membrane 166 since the fluid permeable membrane 166 may be formed from a relatively foldable, flimsy, or otherwise easily deformable material. The fluid permeable support 168 may include any material that may wick, absorb, adsorb, or otherwise allow fluid transport of the bodily fluids, such as any of the fluid permeable membrane materials disclosed herein above. For example, the fluid permeable membrane material(s) may be utilized in a more dense or rigid form than in the fluid permeable membrane 166 when used as the fluid permeable support 168. The fluid permeable support 168 may be formed from any fluid permeable material that is less deformable than the fluid permeable membrane 166. For example, the fluid permeable support 168 may include a porous polymer (*e*.*g*., nylon, polyester, polyurethane, polyethylene, polypropylene, etc.) structure or an open cell foam, such as spun nylon fiber. In some examples, the fluid permeable support 168 may include a nonwoven material. In some examples, the fluid permeable support 168 may be formed from a natural material, such as cotton, wool, silk, or combinations thereof. In such examples, the material may have a coating to prevent or limit absorption of fluid into the material, such as a water repellent coating. In some examples, the fluid permeable support 168 may be formed from fabric, felt, gauze, or combinations thereof..

In some examples, the fluid permeable membrane 166 may be optional. For example, the porous material 109 may include only the fluid permeable support 168 (as shown in **FIG. 2D****).** In some examples, the fluid permeable support 168 may be optionally omitted from the porous material 109. For example, the porous material 109 may only include the fluid permeable membrane 166. In an embodiment, the porous material 109 may include a layer other than or in addition to at least one of the fluid permeable membrane 166 or the fluid permeable support 168.

In an embodiment, at least a portion of the porous material 109 (*e.g.,* one or more of the fluid permeable membrane 166 or, more preferably, the fluid permeable support 168) may be hydrophobic. The porous material 109 may be hydrophobic when the porous material 109 exhibits a contact angle with water (a major constituent of bodily fluids) that is greater than about 90°, such as in ranges of about 90° to about 120°, about 105° to about 135°, about 120° to about 150°, about 135° to about 175°, or about 150° to about 180°. The hydrophobicity of the porous material 109 may limit absorption, adsorption, and solubility of the bodily fluids in the porous material 109 thereby decreasing the amount of bodily fluids held in the porous cartridge 104. In an embodiment, at least a portion of the porous material 109 is hydrophobic or hydrophilic. In an embodiment, the fluid permeable support 168 is more hydrophobic (*e*.*g*., exhibits a larger contact angle with water) than the fluid permeable membrane 166. The lower hydrophobicity of the fluid permeable membrane 166 may help the porous material 109 receive the bodily fluids from the urethral opening while the hydrophobicity of the fluid permeable support 168 limits the bodily fluids that are retained in the porous material 109.

The porous material 109 may include at least one inner surface 172 (*e.g.,* inner surface 172 of the fluid permeable support 168) that defines a hollowed region 174. The hollowed region 174 is configured to receive at least a portion of the arm 110. For example, the hollowed region 174 may exhibit a size and shape that generally corresponds to the size and shape of the arm 110 such that the porous material 109 may be slide onto the arm 110. In some embodiments, the hollowed region 174 may exhibit a size that is slightly smaller than the size of the arm 110. In such embodiments, sliding the porous material 109 onto the arm 110 may cause the porous material 109 elastically deform and press against the arm 110 which may help secure the porous material 109 to the arm 110.

As previously discussed, the porous material 109 may exhibit a variety of shapes since the porous material 109 is not disposed in a fluid impermeable barrier. In an example, the porous material 109 may exhibit a generally circular cross-sectional shape *(e.g.,* a generally cylindrical shape). In an example, the porous material 109 may exhibit a generally oblong cross-sectional shape. In an example, the porous material 109 may exhibit a cross-sectional shape including an apex 176, such as a generally triangular cross-sectional shape or a generally circular cross-sectional shape that is pitched on one or more sides. The apex 176 may facilitate insertion of the porous material 109 between the labia folds of the individual which may help secure the fluid collection system 100 to the vaginal region of the individual. The apex 176 may allow the porous material 109 to be positioned adjacent to *(e.g.,* abut) the urethral opening which may decrease the likelihood that bodily fluids leak from the fluid collection system 100.

In some embodiments, as previously discussed, the porous material 109 is not disposed within a fluid impermeable material which may allow the porous material 109 to more effectively remove bodily fluids than at least some conventional fluid collection assemblies. For example, conventional fluid collection assemblies may include a fluid impermeable body enclosing significant portions of the porous materials thereof which limits the percentage of the porous materials thereof that may contact the vaginal region. The fluid collection systems disclosed herein may not include a fluid impermeable material disposed on the porous cartridge 104 except for limited contact between the porous material 109 and the bulge and the sump 122. As such, the porous material 109 is free to contact more of the vaginal region of the individual than the porous materials of conventional fluid collection systems which may make the individual feel drier after urinating.

In some embodiments, not shown, the porous material 109 may include a fluid impermeable layer disposed on one or more portions thereof. The fluid impermeable layer may be disposed on portions of the porous material 109 that are unlikely to contact the vaginal region of an individual during use. The fluid impermeable layer may prevent bodily fluids from leaking from such portions of the porous material 109 and may direct the vacuum towards portions of the porous material 109 that are more likely to contact the vaginal region.

In some embodiments, not shown, the porous material 109 may include one or more inner supports disposed therein that are configured to provide structure to the porous material 109. The one or more inner support may also facilitate securement of the porous material 109, for example, by forming an interference fit with at least one of the body 106 or the connector piece 108.

The fluid collection system 100 illustrated in **FIGS. 1A-1E** is merely one example of a fluid collection system. For example, **FIGS. 2A to 2C** are isometric back, isometric front, and back plan views of a fluid collection system 200, according to an embodiment. **FIG. 2D** is a cross-sectional view of the fluid collection system 200 taken along plane 2D-2D illustrated in **FIG. 2A****.** **FIG. 2E** is an exploded view of the fluid collection system 200. Except as otherwise disclosed herein, the fluid collection system 200 is the same or substantially similar to any of the fluid collection systems disclosed herein. For example, the fluid collection system 200 may include a fluid collection assembly 202 and a porous cartridge 204 reversibly attached to the fluid collection assembly 202. The fluid collection assembly 202 may include a body 206 and a connector piece 208. The porous cartridge 204 may include at least one porous material 209. The fluid collection system 200 also includes an arm 210 that may form part of the fluid collection assembly 202 or the porous cartridge 204.

As previously discussed, the flanges may extend a variety of distances from the central portion 220. The distances that the flanges extend from the central portion 220 may depend on the individual's preference and what the individual finds comfortable. In some embodiments, some individuals may find the bottom flange 230 to be comfortable and, in such embodiments, the bottom flange 230 may extend an significant distance from the central portion 220, as illustrated in **FIGS. 1A-1E****.** In some embodiments, some individuals find that the bottom flange 230 to be more uncomfortable than the other flanges. In such embodiments, the bottom flange 230 may extend from the central portion 220 by a distance that is less than the other flanges (as shown in **FIGS. 2A-2E****)** or the bottom flange 230 may be omitted completely.

As previously discussed, the arm 210 includes one or more perforations 254 formed in one or more arm wall 248 and that the perforations 254 may exhibit a variety of shapes. In an embodiment, as illustrated, the perforations 254 may form a lattice structure that are substantially similar to the one or more elongated slits illustrated in **FIGS. 1D** and **1E** except that the elongated slits are interrupted by one or more generally horizontal beams 277. The horizontal beams 277 may provide additional structure (*e*.*g*., rigidness and/or strength) to the arm 210.

The porous material 209 exhibits a lateral dimension (*e*.*g*., diameter) that varies. In an embodiment, the porous material 209 includes an upper region 278 and a bottom region 280. The upper region 278 and the bottom region 280 may exhibit lateral dimensions that are different. For example, the bottom region 280 includes portions of the porous material 209 that are configured to be positioned in the sump 222 of the fluid collection assembly 202. As such, the bottom region 280 may exhibit a first lateral dimension that allows the bottom region 280 to be positioned within the sump 222 of the fluid collection assembly 302. The upper region 278 may include portions of the porous material 209 that are not positioned within the sump 222. As such, the upper region 278 may exhibit a second lateral dimension that is different than the first lateral dimension of the bottom region 280 since the second lateral dimension of the upper region 278 is not constrained by the dimensions of the sump 222. For instance, as illustrated, the upper region 278 may exhibit a second lateral dimension that is greater than the first lateral dimension. In another instance, the upper region 278 may exhibit a second lateral dimension that is less than the first lateral dimension. As such, the upper and bottom regions 278, 280 of the porous material 209 allows the fluid collection assembly 202 to be used with porous materials exhibiting a variety of thicknesses. The porous material 209 may be compressed during use and the wider upper region 278 relative to the bottom region 280 may also accommodate such compression. For example, if the upper region 278 was not wider than the bottom region 280, the compression of the porous material 209 may form a gap between the porous material 209 and the sump 222. Bodily fluids may leak through the gap. The wider upper region 278 prevents or at least inhibits the formation of such gaps. The wider upper region 278 relative to the bottom region 280 may also make the fluid collection system 200 more comfortable. For example, the wider upper region 278 may at least partially prevent the formation of a step between the porous material 209 and the sump 222 that may uncomfortable press the sump 222 into the vaginal region of the individual.

In an embodiment, not shown, the porous material 209 may exhibit cross-sectional shapes that vary. For example, the bottom region 280 may exhibit a first cross-sectional shape that corresponds to the cross-sectional shape of the sump 222 thereby allowing the bottom region 280 to be positioned in the sump 222. However, the upper region 278 may exhibit a second cross-sectional shape that is different than the cross-sectional shape of the sump 222. As such, the upper and bottom regions 278, 280 of the porous material 209 allows the fluid collection assembly 202 to be used with porous materials exhibiting a variety of cross-sectional shapes.

As previously discussed, the arm of the fluid collection system may form part of the fluid collection assembly. **FIG. 3** is a partially exploded view of a fluid collection system 300 illustrating the fluid collection assembly 302 and the porous cartridge 304 spaced from each other, according to an embodiment. Except as otherwise disclosed herein, the fluid collection system 300 is the same as or substantially similar to any of the fluid collection systems disclosed herein. For example, the fluid collection assembly 302 include a body 306 and a connector piece 308. The porous cartridge 304 includes at least one porous material 309.

The fluid collection system 300 include an arm 310. The arm 310 forms part of the fluid collection assembly 302. For example, the bottom portion (not shown, obscured) of the arm 310 may be disposed in the sump 322 of the body 306. The bottom portion of the arm 310 may be attached (*e*.*g*., non-reversibly attached) to at least one of the sump 322 or another component of the fluid collection assembly 302. For example, the bottom portion of the arm 310 may be attached to the sump 322 or another component of the fluid collection assembly 302 using an adhesive, stitches, an interference fit, or any other suitable attachment.

The arm 310 is configured to reversibly connect the porous cartridge 304 to the fluid collection assembly 300. For example, as previously discussed, the porous material 309 may define a hollowed region (*e.g.,* the hollowed region 174 shown in **FIG. 1D****).** The porous cartridge 304 may be attached to the arm 310 by disposing the top portion 356 of the arm 310 adjacent to the opening of the hollowed region and sliding the arm walls 348 of the arm 310 into the hollowed region. The arm flanges (*e.g.,* the arm flanges 158 shown in **FIG. 1E****)** prevent over insertion of the porous material 309 on the arm 310. It is noted that inserting the arm 310 into the hollowed region may also position a portion of the porous cartridge 304 into the sump 322 which, via an interference fit, may help secure the porous cartridge 304 to the fluid collection assembly 302. The porous cartridge 304 may then be detached from the fluid collection assembly 302 by sliding the arm 310 out of the hollowed region of the porous material 309.

As previously discussed, the arm of the fluid collection system may form part of the porous cartridge. **FIG. 4** is a partially exploded view of a fluid collection system 400 illustrating the fluid collection assembly 402 and the porous cartridge 404 spaced from each other, according to an embodiment. Except as otherwise disclosed herein, the fluid collection system 400 is the same as or substantially similar to any of the fluid collection systems disclosed herein. For example, the fluid collection system 400 includes the fluid collection assembly 402 include the body 406 and the connector piece 408. The fluid collection system 400 also include the porous cartridge 404 that includes at least one porous material 409.

The fluid collection system 400 include an arm 410. The arm 410 forms part of the porous cartridge 409. For example, the arm walls (not shown, obscured) of the arm 410 may be disposed in the hollowed region of the porous material 409. The arm 410 may be attached to the porous material 409 using any suitable technique. For example, the arm 410 may be adhesively attached to porous material 409, sewn to the porous material 409, form an interference fit with the porous material when disposed in the hollowed region thereof, or include barbs (*e*.*g*., circumferentially extending hose barbs) that attach the arm 410 to the porous material 409. It is noted that the arm flanges 458 of the arm 410 may prevent over insertion of the porous material 409 on the arm 410 when assembling the porous cartridge 404.

In an embodiment, the arm 410 may be configured to reversibly connect the porous cartridge 404 to the fluid collection assembly 400 by disposing the bottom portion 152 of the arm 410 in the sump 422 of the fluid collection assembly 402 thereby reversibly attaching the porous cartridge 404 to the fluid collection assembly 402. For example, disposing the bottom portion 152 in the sump 422 may attach the porous cartridge 404 to the fluid collection assembly 402 by forming an interference fit between the bottom portion 452 and the sump 422. It is noted that inserting the bottom portion 452 of the arm 410 into the sump 422 may also position a portion of the porous material 409 into the sump 422 which, via an interference fit, may help secure the porous cartridge 404 to the fluid collection assembly 402. The porous cartridge 404 may then be detached from the fluid collection assembly 402 by removing the bottom portion 452 of the arm 410 out of the sump 422.

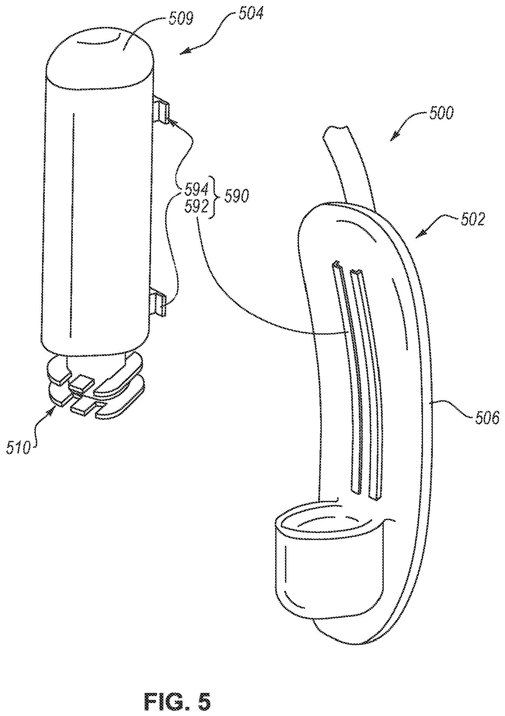

The fluid collection systems disclosed above use the arm to reversibly attach the porous cartridge to the fluid collection system. However, it is noted that the fluid collection systems may include one or more anchors that are distinct from the arm to reversibly attach the porous cartridge to the fluid collection systems. **FIG. 5** is a partially exploded view of a fluid collection system 500 including one or more anchors 590, according to an embodiment. Except as otherwise disclosed herein, the fluid collection system 500 may be the same as or substantially similar to any of the fluid collection systems disclosed herein. For example, the fluid collection system 500 may include a fluid collection assembly 502 including a body 506 and a connector piece (not shown, obscured). The fluid collection system 500 may also include at least one porous cartridge 504 reversibly attached to the fluid collection assembly 502. The porous cartridge 504 includes at least one porous material 509. The fluid collection system 500 may also include an arm 510 that forms part of one of the fluid collection assembly 502 or the porous cartridge 504.

The anchors 590 of the fluid collection system 500 are configured to reversibly attach the fluid collection assembly 502 and the porous cartridge 504 together. In an embodiment, the anchors 590 includes at least one first anchor 592 and at least one second anchor 594 that are configured to be reversibly attached together. The first anchor 592 may be attached to or form part of the fluid collection assembly 502 *(e.g.,* the body 506) and the second anchor 594 may be attached to or form part of the porous cartridge *504 (e.g.,* the porous material 509). It is noted that the anchors 590 may only include a single anchor attached to or integrally formed with the fluid collection assembly 502 or the porous cartridge 504 or may include three or more anchors.

In a particular example, as illustrated, the first anchor 592 is a T-track defining a channel and the second anchor 594 includes a head attached to a shaft, wherein the head of the second anchor 594 is configured to fit in and slide along the channel of the T-track. In such an example, the first and second anchors 592, 594 are attached together by disposing the head of the second anchor 594 in the channel of the first anchor 592 and sliding the head along the channel. It is noted that the anchor 590 may include other anchors other than the anchors 590 illustrated in **FIG. 5****.** In an example, the anchors include one or more magnets and at least one of one or more ferromagnetic materials or oppositely poled magnets. In such an example, one of the fluid collection assembly 502 or the porous cartridge 504 include the magnetic disposed therein or attached thereto and the other of the fluid collection assembly 502 or the porous cartridge 504 include the ferromagnetic materials and/or the oppositely poled magnets disposed therein or attached thereto. In an example, the anchor 590 may include a weak adhesive disposed on surface(s) of one or both of the fluid collection assembly 502 or the porous cartridge 504. In an example, the anchor 590 may include snaps, a hook-and-loop fastener, a string, a clamp, tape, another suitable fastener, or combinations thereof.

In an embodiment, the arm 510 is configured to secure the porous cartridge 404 to the fluid collection assembly 402 in addition to the anchors 590. In an embodiment, the arm 510 is not configured to secure the porous cartridge 504 to the fluid collection assembly 502. In such an embodiment, the bottom portion 552 may be omitted or does not form an attachment with the sump 522, the arm 510 is omitted, or the arm 510 otherwise does not secure the porous cartridge 504 to the fluid collection assembly 502.

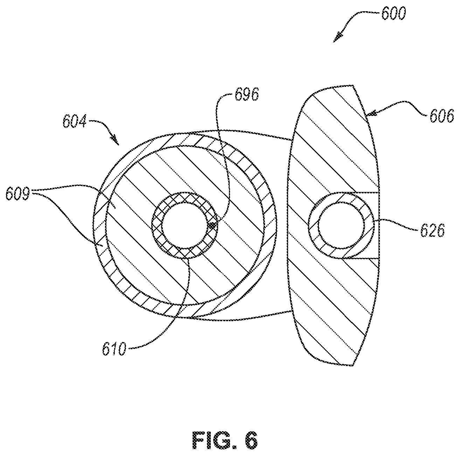

In some embodiments, it may be beneficial to bend at least the porous cartridge such that the porous cartridge conforms to the shape of the vaginal region. Conforming the porous cartridge to the shape of the vaginal region may increase the percentage of bodily fluids that are received into the porous material and inhibit leakage of bodily fluids from the porous material. Contact between the thighs of the individual and the fluid collection system may be used to maintain the bend in the porous cartridge. However, such contact may be difficult to maintain when the individual is skinny or moves. As such, in some embodiments, the fluid collection systems disclosed herein may include a shape memory material that is configured to maintain the shape of the porous cartridge. **FIG. 6** is a cross-sectional view of a fluid collection system 600 taken perpendicularly to a longitudinal axis thereof, according to an embodiment. The fluid collection system 600 includes at least one shape memory material 696 that is configured to maintain a desired shape of the porous cartridge 604. Except as otherwise disclosed herein, the fluid collection system 600 is the same as or substantially similar to any of the fluid collection systems disclosed herein. For example, the fluid collection system 600 may include a fluid collection assembly 602 and a porous cartridge 604. The fluid collection assembly 602 may include a body 606 and a connector piece (not shown). The porous cartridge 604 includes at least one porous material 609. The fluid collection system 600 also includes an arm 610 that may form part of the fluid collection assembly 602 or the porous cartridge 604.

The shape memory material 696 is sized, shaped, and positioned in the fluid collection system 600 to cause at least the porous cartridge 604 *(e.g.,* the porous material 609) to retain a selected shape, such as a shape that corresponds to the shape of the vaginal region. The shape memory material 696 is configured to be bent, shaped, or otherwise deformed (hereafter collectively referred to as "shape," "shaped," or "shaping"). The shape memory material 696 may be more rigid and/or resilient that another portion of the fluid collection system 600 (*e.g.,* the porous material 609) thereby causing at least a portion of the fluid collection system 600 to correspond to the selected shape of the shape memory material 696.

The shape memory material 696 may include at least one polymer and/or at least one metal. Generally, the shape memory materials are composed to adopt an intermediate or permanent shape in response to a stimuli. The stimuli may include an eternal physical force (*e*.*g*., bending force), heat, electrical bias, or a magnetic field. While the term "shape memory material" is used to describe some of the "shape memory materials" herein, it should be understood that, in some examples, the material modified by the term "shape memory material" may not necessarily need to return to a preselected shape upon application of a stimuli, as understood as the classical definition of the "shape memory material." Rather, at least some of the shape memory material disclosed herein may simply hold a selected shape when bent, set, or cured into a specific shape and/or when cooled in a specific shape, regardless of the stimuli applied thereto after. The shape memory materials may be returned to the original shape or changed into a new shape by application of stimuli. For example, a metal wire bent for a first shape may be utilized as the shape memory material whereinafter the metal wire may be modified to a second shape via physical force applied thereto or via heating. However, in some embodiments, the shape memory material may exhibit a selected shape and application of certain stimuli may cause the shape memory material to deform (*e*.*g*., elastically deform or bend) into an intermediate shape. In such embodiments, the shape memory material may return to the initial shape upon removal of the stimuli such that the shape memory material does not maintain the intermediate shape.

In an embodiment, the shape memory material may include one or more metals, such as an elemental metal, an alloy, or shape memory alloy. Suitable shape memory metals may include aluminum, silver, copper, iron, nickel, zinc, tin, beryllium, steel (*e.g.,* carbon steel, heat treated steel, stainless steel), nickel-titanium alloys (*e.g.,* nitinol), copper-based alloys (*e*.*g*., Cu-Zn-Al, Cu-Al-Ni, and Cu-Al-Sn), any other suitable metal, alloys thereof, or combinations thereof. In an embodiment, the shape memory material may include one or more polymers. Suitable shape memory polymers may include polyurethane-based shape memory polymers, thermoplastic polymers, polynorbonene, any other suitable polymer, or combinations thereof.

In an embodiment, as shown, the shape memory material 696 may be disposed in, attached to, or incorporated into the arm 610. For example, as illustrated, the shape memory material 696 may be a wire disposed in the arm 610. The shape memory material 696 may extend along the all of, substantially all of, or a portion of the length of the shape memory material arm 610. The shape memory material 696 may be formed to and maintain a selected shape. The shape memory material 696 may cause the arm 610 to exhibit a shape that generally conforms to the selected shape of the shape memory material 696. The arm 610 may also cause the porous material 609 to exhibit a shape that corresponds to the shape of the arm 610 and the shape memory material 696 because, during use, the arm 610 is disposed in the porous material 609.

The shape memory material 696 may be disposed in other components of the fluid collection system 600 instead of or in addition to the arm 610. For example, the shape memory material 696 may be disposed in, attached to, or incorporated into the body 606 (*e.g.,* in the flanges, the bulge, the sump, etc.), the conduit 626, or the porous material 609 instead of or in addition to disposing, attaching, or incorporating the shape memory material 696 into the arm 610. It is noted that the arm 610 has a larger effect on the shape of the porous material 609 and the porous cartridge 604 as a whole than the body 606 and the conduit 626. As such, the shape memory material 696 of the arm 610 may better control the shape of the porous material 609 than if the shape memory material 696 was disposed in the body 606 or the conduit 626. Also, if the shape memory material 696 becomes detached from the arm 610, the detached shape memory material 696 is less likely to protrude out of the porous material 609 than if the shape memory material 696 forms part of and becomes detached from the porous material 609.

Additional examples of shape memory materials that may be used in the fluid collection systems disclosed herein are disclosed in PCT International Application No. PCT/US2020/042262 filed on July 16, 2020 and U.S. Patent Application No. 17/451,354 filed on October 19, 2021.

The fluid collection systems disclosed herein may include a plurality of porous cartridges. The plurality of porous cartridges allows a used porous cartridge (*e.g.,* a porous cartridge that has received bodily fluids) to be replaced with a new porous cartridge. This allows the fluid collection assemblies disclosed herein to be reusable. In an embodiment, at least some of the plurality of porous cartridges of the fluid collection system are substantially the same. In such an embodiment, the used porous cartridge may be replaced with a new porous cartridge that is substantially the same.

As previously discussed, any of the fluid collection systems disclosed herein may be used with a plurality of porous cartridges. In an embodiment, at least some of the plurality of porous cartridges may be different. For example, the different porous cartridges may exhibit different widths, different lengths, be formed from different materials, or exhibit different cross-sectional shapes. Unlike conventional fluid collection assemblies that may only include a porous material exhibiting a single size, the different porous cartridges allows the individual using the fluid collection assembly to select a porous cartridge that better fits the vaginal region of the individual or select a porous cartridge that the individual finds more comfortable.

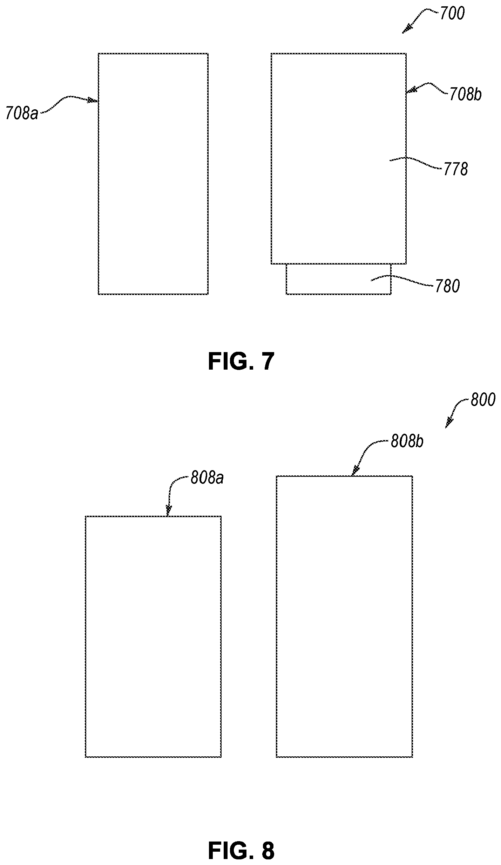

**FIG. 7** is a side plan view of a portion of a fluid collection system 700 that includes a plurality of porous cartridges that include different lateral dimensions, according to an embodiment. Except as otherwise disclosed herein, the fluid collection system 700 and the plurality of porous cartridges are the same or substantially similar to any of the fluid collection systems and porous cartridges disclosed herein. The plurality of porous cartridges includes a first porous cartridge 704a and a second porous cartridge 704b. However, it is noted the fluid collection system 700 may include one or more additional porous cartridges.

The first porous cartridge 704a exhibits a first lateral dimension. The first porous cartridge 704a may exhibit the first lateral dimension along an entire length thereof. The first lateral dimension may be sufficient to fit the first porous cartridge 704a in a sump. The second porous cartridge 704b includes an upper region 778b and a bottom region 780b. The upper region 778b exhibits a second lateral dimension that is different (*e.g.,* greater) than the first lateral dimension. The bottom region 780 exhibits the first lateral dimension which allows the bottom region 780 to fit within the sump. Thus, the first and second porous cartridges 704a, 704b exhibit different lateral dimensions but may be used with the same fluid collection assembly (*e*.*g*., fit within the same sump). The individual using the fluid collection system 700 may select one of the first or second porous cartridge 704a, 704b based on the size of the vaginal region of the individual (*e*.*g*., the individual may select the first porous cartridge 704a or the second porous cartridge 704b if the individual has a relatively small vaginal region or a relatively large vaginal region, respectively) or based on what the individual finds most comfortable.

**FIG. 8** is a side plan view of a portion of a fluid collection system 800 that includes a plurality of porous cartridges that include different lengths, according to an embodiment. Except as otherwise disclosed herein, the fluid collection system 800 and the plurality of porous cartridges are the same or substantially similar to any of the fluid collection systems and porous cartridges disclosed herein. The plurality of porous cartridges includes a first porous cartridge 804a and a second porous cartridge 804b. However, it is noted the fluid collection system 800 may include one or more additional porous cartridges.

The first porous cartridge 804a exhibits a first length. The second porous cartridge 804b exhibits a second length that is different (*e*.*g*., greater) than the first length. The individual using the fluid collection system 800 may select one of the first or second porous cartridge 804a, 804b based on the size of the vaginal region of the individual (*e*.*g*., the individual may select the first porous cartridge 804a or the second porous cartridge 804b if the individual has a relatively small vaginal region or a relatively large vaginal region, respectively) or based on what the individual finds most comfortable.

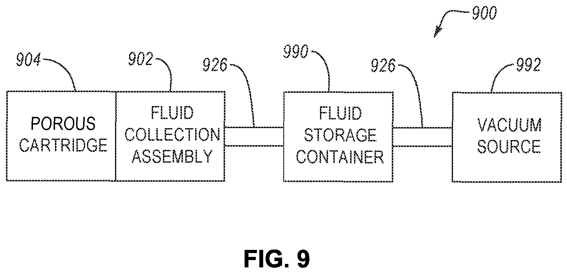

**FIG. 9** is a block diagram of a fluid collection system 900 for fluid collection, according to an embodiment. The fluid collection system 900 includes a fluid collection assembly 902, at least one porous cartridge 904, a fluid storage container 990, and a vacuum source 992. The fluid collection assembly 902 and the porous cartridge 904 may be the same or substantially similar to any of the fluid collection assemblies and porous cartridges 904 disclosed herein. The fluid collection assembly 902, the porous cartridge 904, the fluid storage container 990, and the vacuum source 992 may be fluidly coupled to each other via one or more conduits 926. For example, fluid collection assembly 902 may be operably coupled to one or more of the fluid storage container 990 or the vacuum source 992 via the conduit 926. The bodily fluids collected in the fluid collection assembly 902 and the porous cartridge 904 may be removed from the fluid collection assembly 902 and the porous cartridge 904 via the conduit 926. A vacuum may be introduced into the fluid collection assembly 902 and the porous cartridge 904 via the inlet of the conduit 926 responsive to suction (*e*.*g*., vacuum) force applied at the outlet of the conduit 926.

The vacuum may be applied to the outlet of the conduit 926 by the vacuum source 992 either directly or indirectly. The suction force may be applied indirectly via the fluid storage container 990. For example, the outlet of the conduit 926 may be disposed within the fluid storage container 990 and an additional conduit 926 may extend from the fluid storage container 990 to the vacuum source 992. Accordingly, the vacuum source 992 may apply suction to the fluid collection assembly 902 and the porous cartridge 904 via the fluid storage container 990. The suction force may be applied directly via the vacuum source 992. For example, the outlet of the conduit 926 may be disposed within the vacuum source 992. An additional conduit 926 may extend from the vacuum source 992 to a point outside of the fluid collection assembly 902, such as to the fluid storage container 990. In such examples, the vacuum source 992 may be disposed between the fluid collection assembly 902 and the fluid storage container 990.

The fluid storage container 990 is sized and shaped to retain bodily fluids therein. The fluid storage container 990 may include a bag (*e*.*g*., drainage bag), a bottle or cup (*e*.*g*., collection jar), or any other enclosed container for storing bodily fluids such as urine. In some examples, the conduit 926 may extend from the fluid collection assembly 902 and attach to the fluid storage container 990 at a first point therein. An additional conduit 926 may attach to the fluid storage container 990 at a second point thereon and may extend and attach to the vacuum source 992. Accordingly, a vacuum (*e*.*g*., suction) may be drawn through fluid collection assembly 902 and the porous cartridge 904 via the fluid storage container 990. Bodily fluids, such as urine, may be drained from the fluid collection assembly 902 and the porous cartridge 904 using the vacuum source 992.

The vacuum source 992 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 992 may provide a vacuum or suction to remove bodily fluids from the fluid collection assembly 902 and the porous cartridge 904. In some examples, the vacuum source 992 may be powered by one or more of a power cord (*e*.*g*., connected to a power socket), one or more batteries, or even manual power *(e.g.,* a hand operated vacuum pump). In some examples, the vacuum source 992 may be sized and shaped to fit outside of, on, or within the fluid collection assembly 902. For example, the vacuum source 992 may include one or more miniaturized pumps or one or more micro pumps. The vacuum sources 992 disclosed herein may include one or more of a switch, a button, a plug, a remote, or any other device suitable to activate the vacuum source 992.

While various aspects and embodiments have been disclosed herein, other aspects and embodiments are contemplated. The various aspects and embodiments disclosed herein are for purposes of illustration and are not intended to be limiting.

Terms of degree (*e.g.,* "about," "substantially," "generally," etc.) indicate structurally or functionally insignificant variations. In an example, when the term of degree is included with a term indicating quantity, the term of degree is interpreted to mean ± 10%, ±5%, or +2% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection system, comprising:
a fluid collection assembly (102) including:
a body (106) including a proximal end region (112), a distal end region (114) opposite the proximal end region (112), a front side (116), and a back side (118) opposite the front side (116);
wherein, during use, the distal end region (114) is positioned to be closer to a gluteal cleft than the proximal end region (112) and the front side (116) is positioned to face a vaginal region; and
a connector piece (108) configured to be attached to or integrally formed with the body (106), the connector piece (108) positioned closer to the distal end region (114) of the body (106) than the proximal end region (112), the connector piece (108) defining a conduit connector (136) configured to be connected to a conduit and an arm connector (134), the conduit connector (136) positioned adjacent to the back side (118) of the body (106) and the arm connector (134) positioned adjacent to the front side (116) of the body (106); and
an arm (110) including one or more arm walls (148) defining a cavity (150) and an arm opening configured to be in fluid communication with the arm connector (134), the one or more arm walls (148) defining one or more perforations (154) extending therethrough that allow fluids to flow from an exterior of the arm (110) to the cavity;
**characterised by**
at least one porous cartridge (104) configured to be reversibly attached to the fluid collection assembly (102).

2. The fluid collection system of claim 1, wherein the body (106) includes:
a central portion (120) attached to or integrally formed with the connector piece (108); and
one or more flanges extending from the central portion (120).

3. The fluid collection system of claim 2, wherein the one or more flanges form a generally concavely curve relative to the front side (116) of the body (106).

4. The fluid collection system of any one of claims 1-3, wherein the body (106) of the connector piece (108) defines a sump (122) at or near the distal end region (114) thereof, at least a portion of the arm connector (134) is disposed in the sump (122).

5. The fluid collection system of any one of claims 1-4, wherein the arm (110) includes a top side (156) opposite the arm opening, and wherein the top side (156) includes the one or more perforations (154) formed therein.

6. The fluid collection system of any one of claims 1-5, wherein the arm (110) exhibits a cross-sectional shape exhibiting at least one apex (176).

7. The fluid collection system of any one of claims 1-6, wherein a surface area density of the one or more perforations (154) is greater at or near a top side (156) of the arm (110) than at or near the arm opening.

8. The fluid collection system of any one of claims 1-7, further comprising at least one conduit attached or attachable to the conduit connector (136); and
wherein the body (106) defines a recess (124) that is configured to receive and enclose a portion of the at least one conduit.

9. The fluid collection system of any one of claims 1-8, wherein the at least one porous cartridge (104) including at least one porous material defining a hollowed region (174), the hollowed region (174) configured to have the arm (110) disposed therein, wherein the at least one porous cartridge (104) and the fluid collection assembly (102) are configured to have the at least one porous cartridge (104) removed from the fluid collection assembly (102) substantially without bending any component of the fluid collection assembly (102).

10. The fluid collection system of claim 9, wherein the at least one porous cartridge (104) includes an upper region and a bottom region, the upper region exhibiting a lateral dimension that is greater than the bottom region.

11. The fluid collection system of any one of claims 9 or 10, wherein the at least one porous cartridge (104) includes a plurality of porous cartridges, at least some of the plurality of porous cartridges exhibiting at least one of different lateral dimensions or different lengths.

12. The fluid collection system of any one of claims 9-11, further comprising one or more anchors (590) distinct from the arm (110), the one or more anchors (590) configured to secure the at least one porous material to the fluid collection assembly (102).

13. The fluid collection system of any one of claims 1-12, further comprising at least one shape memory material (696) at least one of disposed in, attached to, or incorporated in the arm (110).

14. A method of using a fluid collection system according to claim 1, **characterised in that** the method comprises the steps of:
reversibly attaching the first porous cartridge (104) to the fluid collection assembly (102), and
removing the first porous cartridge (104) from the fluid collection assembly (102).

15. The method of claim 14, further comprising, after removing the first porous cartridge (104) from the fluid collection assembly (102), reversibly attaching a second porous cartridge to the fluid collection assembly (102).

## Patentansprüche

1. Flüssigkeitssammelsystem, umfassend:
eine Flüssigkeitssammelanordnung (102), die Folgendes beinhaltet:
einen Körper (106) mit einem proximalen Endbereich (112), einem distalen Endbereich (114) gegenüber dem proximalen Endbereich (112), einer Vorderseite (116) und einer Rückseite (118) gegenüber der Vorderseite (116);
wobei während der Verwendung der distale Endbereich (114) so positioniert ist, dass er sich näher an einer Gesäßfalte befindet als der proximale Endbereich (112) und die Vorderseite (116) so positioniert ist, dass sie einem Vaginalbereich zugewandt ist; und
einen Verbinderteil (108), der dazu konfiguriert ist, an dem Körper (106) angebracht oder einstückig mit diesem ausgebildet zu werden, wobei der Verbinderteil (108) näher an dem distalen Endbereich (114) des Körpers (106) als an dem proximalen Endbereich (112) positioniert ist, wobei der Verbinderteil (108) einen Leitungsverbinder (136) definiert, der dazu konfiguriert ist, mit einer Leitung und einem Armverbinder (134) verbunden zu werden, wobei der Leitungsverbinder (136) benachbart zu der Rückseite (118) des Körpers (106) positioniert ist und der Armverbinder (134) benachbart zu der Vorderseite (116) des Körpers (106) positioniert ist; und
einen Arm (110), der eine oder mehrere Armwände (148), die einen Hohlraum (150) definieren, und eine Armöffnung beinhaltet, die konfiguriert ist, um in Fluidkommunikation mit dem Armverbinder (134) zu stehen, wobei die eine oder die mehreren Armwände (148) eine oder mehrere Perforationen (154) definieren, die sich dadurch erstrecken, die es Flüssigkeiten ermöglichen, von einer Außenseite des Arms (110) zu dem Hohlraum zu fließen;
**gekennzeichnet durch**
mindestens eine poröse Kartusche (104), die dazu konfiguriert ist, reversibel an der Flüssigkeitssammelanordnung (102) angebracht zu werden.

2. Flüssigkeitssammelsystem nach Anspruch 1, wobei der Körper (106) Folgendes beinhaltet:
einen Mittelabschnitt (120), der an dem Verbinderteil (108) angebracht oder einstückig mit diesem ausgebildet ist; und
einen oder mehrere Flansche, die sich von dem Mittelabschnitt (120) erstrecken.

3. Flüssigkeitssammelsystem nach Anspruch 2, wobei der eine oder die mehreren Flansche eine im Allgemeinen konkave Kurve relativ zur Vorderseite (116) des Körpers (106) bilden.

4. Flüssigkeitssammelsystem nach einem der Ansprüche 1-3, wobei der Körper (106) des Verbinderteils (108) einen Sumpf (122) an oder in der Nähe seines distalen Endbereichs (114) definiert und mindestens ein Abschnitt des Armverbinders (134) in dem Sumpf (122) angeordnet ist.

5. Flüssigkeitssammelsystem nach einem der Ansprüche 1-4, wobei der Arm (110) eine Oberseite (156) gegenüber der Armöffnung beinhaltet und wobei die Oberseite (156) die eine oder die mehreren darin ausgebildeten Perforationen (154) beinhaltet.

6. Flüssigkeitssammelsystem nach einem der Ansprüche 1-5, wobei der Arm (110) eine Querschnittsform aufweist, die mindestens einen Scheitelpunkt (176) aufweist.

7. Flüssigkeitssammelsystem nach einem der Ansprüche 1-6, wobei eine Oberflächendichte der einen oder der mehreren Perforationen (154) an oder in der Nähe einer Oberseite (156) des Arms (110) größer ist als an oder in der Nähe der Armöffnung.

8. Flüssigkeitssammelsystem nach einem der Ansprüche 1-7, ferner umfassend mindestens eine Leitung, die an dem Leitungsverbinder (136) angebracht oder anbringbar ist; und
wobei der Körper (106) eine Aussparung (124) definiert, die dazu konfiguriert ist, einen Abschnitt der mindestens einen Leitung aufzunehmen und zu umschließen.

9. Flüssigkeitssammelsystem nach einem der Ansprüche 1-8, wobei die mindestens eine poröse Kartusche (104) mindestens ein poröses Material enthält, das einen ausgehöhlten Bereich (174) definiert, wobei der ausgehöhlte Bereich (174) so konfiguriert ist, dass der Arm (110) darin angeordnet ist, wobei die mindestens eine poröse Kartusche (104) und die Flüssigkeitssammelanordnung (102) so konfiguriert sind, dass die mindestens eine poröse Kartusche (104) aus der Flüssigkeitssammelanordnung (102) entfernt werden kann, im Wesentlichen ohne eine Komponente der Flüssigkeitssammelanordnung (102) zu verbiegen.

10. Flüssigkeitssammelsystem nach Anspruch 9, wobei die mindestens eine poröse Kartusche (104) einen oberen Bereich und einen Bodenbereich aufweist, wobei der obere Bereich eine seitliche Abmessung aufweist, die größer ist als der Bodenbereich.

11. Flüssigkeitssammelsystem nach einem der Ansprüche 9 oder 10, wobei die mindestens eine poröse Kartusche (104) eine Vielzahl von porösen Kartuschen beinhaltet, wobei mindestens einige der Vielzahl von porösen Kartuschen mindestens eine von unterschiedlichen seitlichen Abmessungen oder unterschiedlichen Längen aufweisen.

12. Flüssigkeitssammelsystem nach einem der Ansprüche 9-11, umfassend ferner einen oder mehrere Anker (590), die vom Arm (110) getrennt sind, wobei der eine oder die mehreren Anker (590) konfiguriert sind, um das mindestens eine poröse Material an der Flüssigkeitssammelanordnung (102) zu sichern.

13. Flüssigkeitssammelsystem nach einem der Ansprüche 1-12, ferner umfassend mindestens ein Formgedächtnismaterial (696), das entweder im Arm (110) angeordnet, daran angebracht oder in diesen integriert ist.

14. Verfahren zur Verwendung eines Flüssigkeitssammelsystems nach Anspruch 1, **dadurch gekennzeichnet, dass** das Verfahren die folgenden Schritte umfasst:
reversibles Anbringen der ersten porösen Kartusche (104) an der Flüssigkeitssammelanordnung (102) und
Entfernen der ersten porösen Kartusche (104) aus der Flüssigkeitssammelanordnung (102).

15. Verfahren nach Anspruch 14, ferner umfassend, nach Entfernen der ersten porösen Kartusche (104) aus der Flüssigkeitssammelanordnung (102), reversibles Anbringen einer zweiten porösen Kartusche an der Flüssigkeitssammelanordnung (102).

## Revendications

1. Système de collecte des fluides comprenant :
un ensemble de collecte des fluides (102) comprenant :
un corps (106) comprenant une région d'extrémité proximale (112), une région d'extrémité distale (114) opposée à la région d'extrémité proximale (112), une face avant (116) et une face arrière (118) opposée à la face avant (116) ;
dans lequel, pendant l'utilisation, la région d'extrémité distale (114) est positionnée de manière à être plus proche d'un sillon interfessier que la région d'extrémité proximale (112) et la face avant (116) est positionnée de manière à faire face à une région vaginale ; et
une pièce de connexion (108) configurée pour être attachée ou formée intégralement avec le corps (106), la pièce de connexion (108) étant positionnée plus près de la région d'extrémité distale (114) du corps (106) que de la région d'extrémité proximale (112), la pièce de connexion (108) définissant un connecteur de conduit (136) configuré pour être connecté à un conduit et un connecteur de bras (134), le connecteur de conduit (136) étant positionné adjacent à la face arrière (118) du corps (106) et le connecteur de bras (134) étant positionné adjacent à la face avant (116) du corps (106) ; et
un bras (110) comprenant une ou plusieurs parois de bras (148) définissant une cavité (150) et une ouverture de bras configurée pour être en communication fluidique avec le connecteur de bras (134), la ou les parois de bras (148) définissant une ou plusieurs perforations (154) s'étendant à travers elles qui permettent aux fluides de s'écouler d'un extérieur du bras (110) vers la cavité ;
**caractérisé par**
au moins une cartouche poreuse (104) configurée pour être fixée de manière réversible à l'ensemble de collecte des fluides (102).

2. Système de collecte des fluides selon la revendication 1, dans lequel le corps (106) comprend :
une partie centrale (120) fixée à la pièce de connexion (108) ou formée intégralement avec elle ; et
une ou plusieurs brides s'étendant à partir de la partie centrale (120).

3. Système de collecte des fluides selon la revendication 2, dans lequel la ou les brides forment une courbe généralement concave par rapport à la face avant (116) du corps (106).

4. Système de collecte des fluides selon l'une quelconque des revendications 1 à 3, dans lequel le corps (106) de la pièce de connexion (108) définit un réservoir (122) au niveau ou à proximité de son extrémité distale (114), au moins une partie du connecteur de bras (134) étant disposée dans le réservoir (122).

5. Système de collecte des fluides selon l'une quelconque des revendications 1 à 4, dans lequel le bras (110) comprend une face supérieure (156) opposée à l'ouverture de bras, et dans lequel la face supérieure (156) comprend une ou plusieurs perforations (154) formées à l'intérieur.

6. Système de collecte des fluides selon l'une quelconque des revendications 1 à 5, dans lequel le bras (110) présente une forme de section transversale avec au moins un sommet (176).

7. Système de collecte des fluides selon l'une quelconque des revendications 1 à 6, dans lequel la densité de la surface d'une ou plusieurs perforations (154) est plus grande sur ou près d'un côté supérieur (156) du bras (110) que sur ou près de l'ouverture de bras.

8. Système de collecte des fluides selon l'une quelconque des revendications 1 à 7, comprenant en outre au moins un conduit fixé ou pouvant être fixé au connecteur de conduit (136) ; et
dans lequel le corps (106) définit une cavité (124) configurée pour recevoir et enfermer une partie de l'au moins un conduit.

9. Système de collecte des fluides selon l'une quelconque des revendications 1 à 8, dans lequel l'au moins une cartouche poreuse (104) comprend au moins un matériau poreux définissant une région creuse (174), la région creuse (174) configurée pour avoir le bras (110) disposé à l'intérieur, dans lequel l'au moins une cartouche poreuse (104) et l'ensemble de collecte des fluides (102) sont configurés pour que l'au moins une cartouche poreuse (104) soit retirée de l'ensemble de collecte des fluides (102) pratiquement sans plier aucun composant de l'ensemble de collecte des fluides (102).

10. Système de collecte des fluides selon la revendication 9, dans lequel au moins une cartouche poreuse (104) comprend une région supérieure et une région inférieure, la région supérieure présentant une dimension latérale supérieure à la région inférieure.

11. Système de collecte des fluides selon l'une quelconque des revendications 9 ou 10, dans lequel au moins une cartouche poreuse (104) comprend une pluralité de cartouches poreuses, au moins certaines de la pluralité de cartouches poreuses présentant au moins l'une des dimensions latérales différentes ou des longueurs différentes.

12. Système de collecte des fluides selon l'une quelconque des revendications 9 à 11, comprenant en outre un ou plusieurs ancrages (590) distincts du bras (110), le ou les ancrages (590) étant configurés pour fixer l'au moins un matériau poreux à l'ensemble de collecte des fluides (102).

13. Système de collecte des fluides selon l'une quelconque des revendications 1 à 12 comprend en outre au moins un matériau à mémoire de forme (696) disposé, fixé ou incorporé dans le bras (110).

14. Procédé d'utilisation d'un système de collecte des fluides selon la revendication 1, **caractérisée en ce que** le procédé comprend les étapes suivantes :
fixer de manière réversible la première cartouche poreuse (104) à l'ensemble de collecte des fluides (102), et
retirer la première cartouche poreuse (104) de l'ensemble de collecte des fluides (102).

15. Procédé selon la revendication 14, comprenant en outre, après avoir retiré la première cartouche poreuse (104) de l'ensemble de collecte des fluides (102), la fixation réversible d'une seconde cartouche poreuse à l'ensemble de collecte des fluides (102).
